# EUROPEAN PATENT APPLICATION

(11) **EP 4 804 204 A1**
(43) Date of publication of application: **09.09.2026**
(21) Application number: 25161367.5
(22) Date of filing: 03.03.2025
(51) Int. Cl.: G16H 40/63, G06F 40/40, G10L 15/00, G16H 50/20

(54) **AUTOMATED TESTING OF USER INTERFACES**

(71) Applicant: Fresenius Medical Care Deutschland GmbH, 61352 Bad Homburg (DE)
(72) Inventor: Casaña-Eslava, Raul, 61352 Bad Homburg (DE); Fumagalli, Luca, 61352 Bad Homburg (DE); Neri, Luca, 61352 Bad Homburg (DE); Silvestre-Llopís, Jordi, 61352 Bad Homburg (DE)
(74) Representative: Schwarz, Claudia

(57) **Abstract**

The invention pertains to a computer-implemented method for evaluating a user interface (UI) of a medical device. The method involves the collection of sensor data, both textual and non-textual, during the use of the UI, which reflects the user's satisfaction with the interface during interaction. The non-textual sensor data is transformed into textual format, and all received sensor data is aggregated in a textual format. This aggregated data is then input into an assessment algorithm, which continuously determines an assessment dataset. The resulting dataset is then displayed on a result interface. The medical device can be any technical device used for medical purposes, such as a dialysis or plasmapheresis device. The UI can be a variety of interfaces, including a graphical user interface (GUI), a gesture-based interface, a voice interface, or a keyboard, and can be implemented directly in the medical device or in a separate device in data exchange with the medical device. The sensor data can come from various sensors, including text data, voice data, data from a virtual sensor, and image data from an optical sensor.

## Description

The present invention is within the field of medical devices like dialyses devices and relates to a technique for user interface assessment of these devices. In particular the invention relates to a method for determining an assessment for a user interface, UI, of such a medical device, a computing device for determination of such an assessment, a system comprising the computing device, and a computer program product.

When medico-technical devices are operated through graphical user interfaces (GUI) under elevated psychological stress, user-friendliness is key. In medicine, in particular in intensive care, the efficient and foolproof operation can be critical to survival, both in the event of an immediate emergency or when managing the medical condition at home or within a typically busy and underfunded health centre.

In a dialysis centre, for example, a shortage in both medical staff and equipment leads to patients competing for treatment slots at the machines, which are operated by overworked and tired nurses, who in addition may be poorly trained. A peritoneal dialysis (PD) patient at home may be distressed, confused, or distracted when adjusting the device without assistance. Such circumstances increase the risk of unnecessary service errors. A GUI that is intuitive and easy to handle may save time and prevent mistakes, thus alleviating care duties and potentially extending life spans.

In the current state of the art, the assessment of user interfaces (UI) for medical devices is often a manual and subjective process. This can lead to inconsistencies in the evaluation and may not accurately reflect the user's satisfaction with the UI. Furthermore, the process can be time-consuming and inefficient. Additionally, the assessment process may not take into account all types of sensor data, particularly non-textual sensor data, which can provide valuable insights into the user's interaction with the Ul.

In state of the art in other fields of technology it is known to improve UI design. For example, US 9,652,510 B1 describes systems and user interfaces in a non-medical domain that utilize artificial intelligence (Al) algorithms to analyse data and generate optimized packages of data items for television advertising, in which advertising spots (e.g., data items) are matched with advertisers (e.g., another type of data item), prices for the advertising spots are determined, and the advertising spots are packaged together into a deal. The system evaluates various metrics, such as network availability and event demand, to prioritize and score events. It then recommends a subset of events to users based on their preferences and demographics. The goal is to enhance data analysis processes by leveraging Al to provide tailored recommendations and insights.

US 2005/054,381 A1 outlines a system designed to analyse customer behavior patterns. It involves profiling customers to understand their behaviors and preferences, enabling businesses to compare different customer behavior patterns effectively. The system aims to provide insights that can inform marketing strategies, product development, and customer relationship management by identifying trends and patterns in customer actions.

It is therefore an object of the present invention to provide a solution for automatic assessment of user interface interaction, which allows for a more objective, comprehensive, and efficient evaluation process. Further, assessment should be based on actual interaction with the user interface.

This object is solved by a method for determining an assessment for a user interface, UI, of a medical device, by a computing device, a system, by a computer program (and/or computer program product), according to the appended independent claims. Advantageous aspects, features and embodiments are described in the dependent claims and in the following description together with advantages.

The field of this invention is related to the assessment of user interfaces (UI) for medical devices. Medical devices, such as blood treatment devices or dialysis devices, are technical devices used for medical purposes and treatments. These devices comprise medical units and electronic units that primarily serve to control and operate the device. The UI of these devices is a critical component as it allows the medical practitioner or the patient him- or herself to operate the device safely and efficiently. The UI could be a graphical user interface (GUI), a gesture-based interface, a voice interface, or a keyboard, and it could be directly implemented in the medical device or in a separate device in data exchange with the medical device.

The assessment of the UI in particular with its design and its functionality (e.g., when which kind of user prompts are requested etc.) is crucial to ensure the device is user-friendly and efficient. This is typically done by collecting sensor data during the use of the UI. The sensor data, which could be text data, voice data, data from a virtual sensor, or image data from an optical sensor, reflect the user's satisfaction with the UI during the course of interaction. The sensor data could be multi-modal and could be acquired from different sensor types, including complex sensors which comprise software for analyzing the sensor data, like a time-measurement sensor, a gesture sensor for gesture recognition, a wired glove sensor, an acoustic sensor, an input on a user interface, virtual sensor data, and/or an optical sensor.

In the following, the invention will be described with respect to the claimed method first. Features, advantages or alternative embodiments, mentioned with respect to the method can be assigned to the other claimed objects (e.g. the computer program or a device) and vice versa. In other words, the system, or device can be improved with features described or claimed in the context of the method and vice versa. In this case, the functional features of the method are embodied by structural units of the device or system and vice versa, respectively. The method may refer to a software implementation and the device may refer to a hardware implementation. Generally, in computer science a software implementation and a corresponding hardware implementation (e.g. as an embedded system) are equivalent. Thus, for example, a method step for "storing" data may be performed with a storage unit and respective instructions to write data into the storage. For the sake of avoiding redundancy, although the device may also be used in the alternative embodiments described with reference to the method, these embodiments are not explicitly described again for the device. In principle, the respective device is configured to carry out the claimed method.

The invention concerns a method and system for assessing the user interface (UI) of a medical device. This involves the use of sensor data, both textual and non-textual, collected during the use of the UI, i,e., during the course of user interface interaction. The non-textual sensor data is transformed into textual data, and all data is aggregated in particular in the textual format and fed into an assessment algorithm to determine an assessment dataset. The system can comprise various types of sensors, inter alia comprising time-measurement sensors, gesture sensors, acoustic sensors, and/or optical sensors. The assessment algorithm can use different agents for analysis, including a research agent and a critic agent. The system can also use large language models (LLMs) in particular for transforming non-textual data into textual data and/or for providing an assessment result. The described technology can be used in a variety of medical devices, including dialysis devices and other blood treatment devices.

The method for determining an assessment for a UI could be executed as a hidden process simultaneously with or during user interface interaction. The method could automatically stop if the determined assessment dataset does not change or does not provide new information content. The assessment algorithm could also analyze the aggregated data with regard to its semantic content and could use a metrics calculation agent to calculate metrics for text analysis, particularly for classification. The research agent could use a classification technique to assign a label to data points of the aggregated data which serve as input features. The classification technique could include Logistic Regression, Decision Trees, Random Forests, Support Vector Machines, or Neural Networks, particularly deep neural networks (DNN).

The key advantage of this invention is its ability to provide a more objective and comprehensive assessment of the UI. By transforming non-textual sensor data into textual sensor data, the method can incorporate a wider range of user feedback formats and/or data into the assessment, leading to a more accurate reflection of the user's satisfaction with the UI. The use of an assessment algorithm also ensures a consistent and objective evaluation process.

Furthermore, the method as described above may be applied or used by different users of the medical device (like different nurses or medical practitioners or different patients) which allows a more comprehensive and inter-personal evaluation and assessment.

In a preferred embodiment, each or selected ones of the determined assessment datasets are assigned with a user label. The user label represents the identity of the user, evaluating the UI. This has the technical advantage that it is possible to execute statistical algorithms of the assessment datasets with respect to the user's identity. So, for instance, it is possible to evaluate, the consecutive assessments of the user for the UI over time. Further, it is possible to evaluate assessments of different users and compare them and/or determine an average assessment.

Alternatively or in addition, a location label may be attached to the assessment dataset. The location label represents the geographical location where the UI of the medical device is located. With this feature, it is possible to execute an analyses where and in particular in which medical units which user feedback (in the form of sensor data) is acquired.

Furthermore, the method enhances efficiency by automating the assessment process. This can save time and resources compared to manual assessment methods. The method also allows for continuous determination of an assessment dataset, providing real-time feedback on the UI. This can facilitate quicker identification and resolution of any issues with the UI, leading to improved user satisfaction and potentially better patient outcomes.

Further, acoustic user feedback is detected as acoustic sensor signals (e.g, voice recording via microphone). This has the technical advantage that it is very easy and efficient for the user to give her or his feedback during the course of UI interaction. No additional buttons or switches must be activated, the user can simply speak while interacting with the UI.

The invention relates to a computer-implemented method for determining an assessment for a user interface (UI) of a medical device. The medical device can be a technical device used for medical purposes and/or medical treatment, comprising medical units and/or electronic units that primarily serve to control and/or operate the medical device. In a preferred embodiment, the medical device is a blood treatment device, particularly a dialysis device. Other possible medical devices include peritoneal dialysis devices or plasmapheresis devices.

Dialysis is typically used to replace kidney function by removing waste toxins and excess water. Ensuring correct operation during treatment and therefore during operation of the medical device is crucial. This correct operation of the medical device requires correct user interface interaction. Therefore, the design of the user interface is of key importance to ensure that the medical practitioner can operate the medical device quickly, safely, and securely.

The user interface may be a Graphical User Interface (GUI), a gesture-based interface, a voice interface, and/or a keyboard. The user interface may be directly implemented in the medical device or may be implemented in a separate device that exchanges data with the medical device. The user interface may comprise several interface components, like a first component, a second component and/or further components which all together form the user interface. In this case the sensor data refer to the use of at least one or all of the components of the UI.

The method involves receiving sensor data during the use of the user interface. This sensor data may include textual sensor data and non-textual sensor data. The sensor data reflects the user's satisfaction with the user interface during the course of user interface interaction. The user may be the individual actually operating the user interface or a user who is supervising or observing the user interface interaction of an operating user. The sensor data may refer to the user interface or to its components. In the latter case, the sensor data may comprise annotation data. The annotation data represent information with respect to the component of the UI. Annotation data may identify the component of the UI.

The sensor data may come from different sensors, typically but not necessarily operating in parallel. For example, the sensor data may be text data, voice data, data from a virtual sensor that senses the user's selections of a user interface or keyboard or touchboard actions, data that senses user actions with respect to operating the medical device, data that senses the user's gestures, particularly requested head or hand signals, and/or image data from an optical sensor.

In particular, the sensor data may comprise a voice recording. Voice recordings is an audio dataset which represent the user's verbal feedback via voice memo or voice function. For more detailed feedback speech analysis tools may be used, like chatbots collecting additional or more detailed feedback based on spoken questions/answers. The audio data may in a subsequent step be transformed into text data for further processing.

The sensor data may comprise a timestamp or a reference in time so that it is possible to evaluate the sensor data with a time reference by the assessment algorithm. Thus, knowledge exists, when or at what time which user interface element was activated in which manner and associated to this user interface interaction which sensor data are detected. For example, it is possible to assign sensor data in the form of spoken words ("this interaction was efficient and very helpful"), acquired by means of a microphone to the respective user interface element which was used and to which the voice recording refers to.

The sensor data could be raw sensor data, directly originating from a sensor and/or may be processed data, like analog-digitally converted sensor data, or software-based processed sensor data.

The sensor data may be captured on an evaluation UI (another UI as the one being assessed) for requesting the user to give a star rating (e.g., 0 stars for bad evaluation and 5 stars for best evaluation and intermediate evaluations), a numeric evaluation from 0 to 10 or by requesting answers on multiple choice questions on the evaluation UI. The sensor data may be captured as heatmaps. With this tool, it is possible to record where the user clicks, scrolls or pauses in order to identify problematic areas. Sensor data may be captured as video recordings, which capture user interaction and optionally with comments on specific actions.

Alternatively or in addition, the sensor data may be captured as usage data. Usage data may refer to an indirect feedback. Usage data are observations of the user interface interactions. Usage data do not originate from the user directly but refer to the observation of the users interactions. Usage data may relate to evaluations of user behavior without direct input from the user. For instance, usage data may comprise the tracking of clicks, loading times or cancellation rates. Alternatively or in addition, usage data may be provided as heatmaps or session replays.

The method further involves transforming the non-textual sensor data into textual sensor data.

This transformation allows for a more uniform analysis of the sensor data.

The method also involves aggregating all received sensor data, comprising sensor data in different formats, in particular in the textual and non-textual format, in a textual format. This aggregation allows for a comprehensive analysis of the sensor data.

Aggregating (all) received sensor data may comprise aggregating sensor data of different users of the same user interface. Alternatively or in addition, aggregating (all) received sensor data may comprise aggregating sensor data from different user interfaces, which may serve as reference and/or which may be processed by the assessment algorithm.

The aggregated data is then fed into an assessment algorithm. This algorithm continuously (in particular with reference to the user interface interaction and associated therewith acquisition of sensor data) determines an assessment dataset as a result. This assessment dataset provides a comprehensive evaluation of the user's satisfaction with the user interface.

Finally, the method involves providing the determined assessment dataset on a result interface. In one embodiment, this result interface allows for the easy viewing and analysis of the assessment dataset. This can help in making improvements to the user interface, thereby enhancing the user's satisfaction with the user interface. Alternatively or in addition, the assessment dataset may be provided via the result interface (being implemented as a data interface) to downstream applications, e.g., for automatically changing UI programming based on the assessment dataset.

The assessment dataset may comprise an indication of usability. The indication of usability may comprise an indication of intuitiveness, comprising answers to questions like: "Are the functions easy to find and understand?". For example: "I couldn't find the settings immediately." Further, the indication of usability may relate to a navigation structure. The navigation structure of the UI relates to questions like: "Is the navigation structure simple and logical?" For example: "There are too many clicks to complete an action." The indication of usability may relate to accessibility. Accessibility relates to questions like: "Can users with different abilities use the interface?" For example: "The font size is too small for people with visual impairments." Alternatively or in addition the indication of usability may comprise an indication of efficiency. The indication of efficiency may answer questions like: "How quickly can users complete tasks?" For example: "It takes too long to upload data."

Alternatively or in addition the assessment dataset may comprise an evaluation of functionality. The evaluation of functionality may relate to feedback on the technical functionality of the user interface. The evaluation of functionality may comprise an indication of error messages. This may relate to answers to questions like: "Are error messages helpful and understandable?" For example: "The error code did not tell me what to do." The evaluation of functionality may relate to technical stability. This refers to answers to questions like: "Are there problems such as crashes or lags? For example: "The page freezes when I upload a lot of images." Alternatively or in addition, the evaluation of functionality may relate to compatibility. This may relate to answers to questions like: "Does the interface work on different devices and/or browsers? For example: "The mobile version doesn't work properly on my device."

Alternatively or in addition, the assessment dataset may comprise an indication of feedback and interaction flow. This concerns, how the UI interacts with the user and provides feedback. The indication of feedback and interaction flow may comprise feedback on response time, indicating, how quickly does the interface respond to user input. For example: "Loading pages takes too long." Alternatively or in addition, the indication of feedback and interaction flow may relate to clarity of instructions, indicating whether are evaluated as instructions or tooltips helpful and understandable. For example: "The wording in the instructions is confusing." Alternatively or in addition, the indication of feedback and interaction flow may relate to confirmation and success messages, indicating whether there are clear signals that an action was successful? For example: "There is no success message after submitting a form."

Alternatively or in addition, the assessment dataset may comprise indications for personalization and customization. This comprises feedback on the possibility of customising the interface in particular to the user's needs and/or the settings of usages (e.g., medical scenario).

An advantage of the above method is that the assessment of UI interaction may be executed during normal use of the GUI as a process hidden in the background, which does not affect proper functionalities. Thus, the tool may track the user behaviour on the interface, including choices they make or revoke, and the time spent on each of them, when using selection menus, tick-off boxes and the like to control the device. Alternatively or in addition, the assessment includes collecting think-aloud feedback while the user navigates the GUI. Alternatively or in addition, the process may engage the user in two-sided semi-structured interviews, performed verbally, in writing or pictorially, about the GUI's performance.

In a preferred embodiment, a natural stop criterion is implemented to the process. A simple metric function will detect when all cluster sizes have stopped changing. Alternatively or in addition, the method allows the process to be stopped prematurely through outside intervention (e.g., manual input via UI).

Preferably, the result of the assessment is accessible to the user for their consent. Preferably, the result is sufficiently detailed for a developer to conclude on the user's preferences regarding specific GUI features. In one embodiment of the invention, two or more agents feed into a single final report.

Alternatively, or in addition, the method as described above may be executed as an add-on directly of the medical device. Alternatively, or in addition, the method may be executed on an assessment device, with a digital processing unit which is external to the medical device. In particular, the method may be executed on a mobile device with data connection to the medical device, the sensors and optionally to a cloud-based server, which is configured for aggregating the determined assessment datasets in particular for downstream analysis. This has the advantage that the usage of the medical device and its UI is not distracted from the user's assessment.

The method can involve the use of multi-modal sensor data, or data acquired from different types of sensors. These sensors can include a time-measurement sensor, a gesture sensor for gesture recognition, a wired glove sensor, an acoustic sensor (particularly a microphone), input on a user interface, virtual sensor data, and an optical sensor.

The time-measurement sensor can be used to track the duration of certain actions or processes, providing valuable information about the efficiency and ease of use of the user interface. Alternatively or in addition the time-measurement sensor is configured to measure a time between a request providing a request to a user for action and receiving the requested user action. The gesture recognition sensor can recognize specific movements made by the user, such as hand or arm motions, which can be particularly useful in interfaces that rely on gesture-based control. In particular, a kinetic user interface, KUI may be used. A wired glove sensor can provide detailed data about the user's hand movements and finger positions, offering insights into how the user interacts with the interface. Optical sensors, like cameras and associated software may be used to analyze image-based face expression recognition. The sensor is thus not used for user identification but for semantic assessment of facial features.

An acoustic sensor, such as a microphone, can capture voice commands or other audible signals from the user. This can be especially relevant in user interfaces that incorporate voice recognition technology. Inputs on a user interface, such as clicks, taps, or keystrokes, can also be tracked and analyzed to understand the user's interaction with the interface.

Virtual sensor data can include information generated by software-based sensors, which can monitor various aspects of the system's operation or the user's behavior. Finally, an optical sensor can capture visual data, such as the user's eye movements or facial expressions, providing further insights into the user's satisfaction with the user interface.

By utilizing multi-modal sensor data or data from different types of sensors, the method can provide a comprehensive assessment of the user interface, taking into account various aspects of the user's interaction with the interface. This can lead to a more accurate and detailed evaluation, which can in turn inform improvements to the user interface design.

The method can involve the use of an assessment algorithm that may employ a plurality of different agents, including at least a research agent and a critic agent. These agents are configured to analyze the received sensor data to provide an assessment.

The research agent can provide a first result. This agent can be designed to explore the sensor data, identifying patterns, trends, or other notable features. The research agent's analysis can be broad and comprehensive, covering a wide range of potential factors that could influence the user's satisfaction with the user interface. In particular, the research agent may a list of themes and topics based on the received sensor data. Alternatively or in addition the research agent may set up an appropriate classification system for the sensor data received and/or classify the sensor data.

The critic agent can provide a second result. This agent can be designed to evaluate the sensor data more critically, focusing on specific aspects of the data or applying particular criteria or standards in its analysis. The critic agent's analysis can be more targeted and specific, honing in on key areas of concern or interest. In particular, the critic agent may evaluate the completeness of the classification made by the research agent. Alternatively or in addition, the critic agent may evaluate the semantic sensibility of the classification.

A reconciliation algorithm can then be applied to the first and second results. This algorithm can combine, compare, or otherwise reconcile the results provided by the research agent and the critic agent. The reconciliation algorithm can take into account the different perspectives and approaches of the two agents, ensuring that the final result is balanced and comprehensive.

The final result provided by the reconciliation algorithm can represent the overall assessment of the user interface. This result can incorporate the insights and findings of both the research agent and the critic agent, providing a thorough and nuanced evaluation of the user's satisfaction with the user interface. This can help to inform improvements to the user interface, enhancing its usability and effectiveness.

The method can involve the use of an assessment algorithm that is based on a first large language model (LLM). This LLM is trained to provide an assessment result for textual input.

A large language model is a type of machine learning model that is trained on a large amount of text data. The model learns to predict the next word in a sentence based on the words that came before it. This allows the model to generate text that is coherent and contextually appropriate.

In the context of the method, the first LLM can be trained to analyze the textual sensor data and provide an assessment result. The training process can involve exposing the model to a large amount of text data related to user interface interactions and user satisfaction. The model can learn to recognize patterns, trends, and other features in the data that are indicative of the user's satisfaction with the user interface.

Once trained, the first LLM can analyze the textual sensor data received during the use of the user interface. The model can generate an assessment result based on this analysis, providing a measure of the user's satisfaction with the user interface.

By using a first large language model, the method can leverage the power of machine learning to provide a detailed and nuanced assessment of the user interface. This can help to identify areas for improvement and enhance the overall usability and effectiveness of the user interface.

The method can involve the use of a second large language model (LLM) for the transformation step. This second LLM is trained to transform non-textual data into textual data.

In the context of the method, the second LLM can be trained to convert non-textual sensor data into a textual format. The training process can involve exposing the model to a large amount of non-textual data related to user interface interactions and user satisfaction, along with corresponding textual representations of this data. The model can learn to recognize patterns, trends, and other features in the non-textual data and translate these into a textual format.

Once trained, the second LLM can be used to transform the non-textual sensor data received during the use of the user interface into textual sensor data. This transformed data can then be aggregated with the original textual sensor data for further analysis.

By using a second large language model for the transformation step, the method can ensure that the non-textual sensor data is accurately and effectively converted into a textual format. This can facilitate a more comprehensive and nuanced analysis of the sensor data, leading to a more accurate assessment of the user's satisfaction with the user interface.

As already mentioned above, the method can be executed as a hidden process simultaneously with and/or during user interface interaction. This means that the method can run in the background, without disrupting or interfering with the user's interaction with the user interface. The user may not even be aware that the method is being executed.

Executing the method as a hidden process can have several advantages. For one, it allows for real-time or near-real-time analysis of the sensor data, providing immediate feedback on the user's satisfaction with the user interface. This can enable prompt adjustments or improvements to the user interface, enhancing its usability and effectiveness.

Furthermore, running the method as a hidden process can minimize any potential impact on the user's experience. Since the method does not interfere with the user's interaction with the user interface, it does not disrupt the user's workflow or cause any inconvenience. This can help to maintain the user's satisfaction with the user interface, even as the method is being executed.

By executing the method as a hidden process, the method can provide a seamless and unobtrusive way to assess the user's satisfaction with the user interface. This can lead to more accurate and reliable assessment results, contributing to the overall effectiveness of the method.

The method can include an automatic stop feature if the determined assessment dataset does not change or does not provide new information content. This means that the method can cease operation if it determines that the assessment dataset is no longer providing new or useful insights about the user's satisfaction with the user interface.

The determination of whether the assessment dataset is providing new information can be based on an entropy-based algorithm. Entropy is a measure of the randomness or unpredictability of data. In the context of the method, an entropy-based algorithm can analyze the assessment dataset to determine whether it contains new or unpredictable information. If the entropy of the assessment dataset is low, indicating that the data is predictable or repetitive, the method can automatically stop.

Alternatively, or in addition, the determination of whether the assessment dataset is providing new information can be based on testing of the generation of new categories or subcategories. This can involve analyzing the assessment dataset to see if it contains information that falls into new categories or subcategories that were not previously identified. If no new categories or subcategories are being generated, indicating that the data is not providing new insights, the method can automatically stop.

By including an automatic stop feature, the method can avoid unnecessary computation and resource usage when the assessment dataset is no longer providing useful information. This can make the method more efficient and cost-effective, while still ensuring that valuable insights about the user's satisfaction with the user interface are captured.

The method can involve the assessment algorithm analyzing the aggregated data with regard to its semantic content. Semantic analysis refers to the process of understanding the meaning of the data. In the context of the method, this can involve interpreting the textual sensor data to understand the user's satisfaction with the user interface.

Semantic analysis can involve various techniques, such as natural language processing, sentiment analysis, and topic modeling. Natural language processing can be used to understand the structure and meaning of the textual sensor data. Sentiment analysis can be used to determine the user's feelings or attitudes towards the user interface, based on the textual sensor data. Topic modeling can be used to identify the main themes or topics in the textual sensor data.

By analyzing the semantic content of the aggregated data, the assessment algorithm can gain a deeper understanding of the user's satisfaction with the user interface. This can provide more nuanced and detailed insights, leading to a more accurate assessment of the user interface. This can in turn inform improvements to the user interface, enhancing its usability and effectiveness.

The method can involve the assessment algorithm using a metrics calculation agent, which is responsible for calculating metrics for text analysis, particularly for classification. The metrics calculation agent can use coverage, affinity, and relevance to evaluate the quality of the classification.

Coverage refers to the extent to which the classification captures all relevant aspects of the textual sensor data. A high coverage means that the classification includes a wide range of relevant information from the data.

Affinity refers to the degree of match or similarity between the classification and the actual content of the textual sensor data. A high affinity means that the classification closely matches the data.

Relevance refers to the importance or significance of the classification in relation to the user's satisfaction with the user interface. A high relevance means that the classification provides valuable insights into the user's satisfaction.

If the quality of the classification, as determined by the coverage, affinity, and relevance, is below a pre-defined threshold, the method can take appropriate action. This could include adjusting the classification process, re-analyzing the data, or alerting a human operator for further investigation.

By using a metrics calculation agent, the assessment algorithm can ensure that the classification of the textual sensor data is accurate, comprehensive, and relevant. This can lead to a more reliable and meaningful assessment of the user's satisfaction with the user interface.

The method can involve the assessment algorithm comprising a research agent and a critic agent. The research agent is responsible for text classification, while the critic agent is responsible for evaluating the completeness and/or the semantic sensibility of the researcher's classification.

The research agent can analyze the textual sensor data and classify it into various categories or groups based on its content. This classification can provide insights into the user's satisfaction with the user interface, as reflected in the sensor data.

The critic agent can then evaluate the classification provided by the research agent. This evaluation can involve assessing the completeness of the classification, ensuring that all relevant aspects of the sensor data have been captured. The critic agent can also assess the semantic sensibility of the classification, ensuring that the classification makes sense in the context of the sensor data and the user's interaction with the user interface.

By including both a research agent and a critic agent, the assessment algorithm can ensure that the classification of the textual sensor data is both comprehensive and semantically sensible. This can lead to a more accurate and meaningful assessment of the user's satisfaction with the user interface.

The method can involve the research agent using a classification technique to assign a label to data points of the aggregated data, which serve as input features. This classification can help to organize and interpret the data, providing insights into the user's satisfaction with the user interface.

Various classification techniques can be used by the research agent. These can include Logistic Regression, Decision Trees, Random Forests, Support Vector Machines, and Neural Networks, particularly deep neural networks.

Logistic Regression is a statistical method that can predict the probability of a binary outcome based on one or more predictor variables. Decision Trees are a type of model that uses a tree-like graph or model of decisions and their possible consequences. Random Forests are an ensemble learning method that operates by constructing multiple decision trees and outputting the class that is the mode of the classes of the individual trees. Support Vector Machines are supervised learning models that analyze data and recognize patterns, used for classification and regression analysis. Neural Networks are a set of algorithms, modeled loosely after the human brain, designed to recognize patterns.

In particular, deep neural networks, a type of neural network with multiple layers between the input and output layers, can be used. These layers, also known as hidden layers, allow the network to learn complex patterns and relationships in the data.

By using these classification techniques, the research agent can effectively analyze the aggregated data and provide a detailed and nuanced classification. This can lead to a more accurate and meaningful assessment of the user's satisfaction with the user interface.

According to a second aspect, the invention also relates to a computing device for determining an assessment for a user interface of a medical device. The computing device includes a sensor interface for receiving sensor data from sensors during the use of the user interface. The sensors can include virtual sensors, which acquire textual sensor data, and physical sensors, which acquire non-textual sensor data. The sensor data reflects the user's satisfaction with the user interface during the course of user interface interaction.

A virtual sensor can be a software-based sensor, which determines sensor data based on input data, such as spoken or written text. The virtual sensor can alternatively or additionally include at least one optical sensor, comprising Light Detection and Ranging (Lidar), Optical Coherence Tomography (OCT), Photonic Sensors, and/or Camera sensors to acquire images. The images can be analyzed by software to detect the meaning represented in the image, such as a gesture.

The computing device also includes a transformer, which is configured to transform the non-textual sensor data into textual sensor data. This transformation allows for a more uniform analysis of the sensor data.

The computing device further includes an aggregator for aggregating all received sensor data in a textual format. This aggregation allows for a comprehensive analysis of the sensor data.

The computing device also includes a processing unit, which is configured to execute an assessment algorithm with the aggregated data for continuously determining an assessment dataset as a result. This assessment dataset provides a comprehensive evaluation of the user's satisfaction with the user interface.

Finally, the computing device includes a result interface for providing the determined assessment dataset. This result interface allows for the easy viewing and analysis of the assessment dataset. This can help in making improvements to the user interface, thereby enhancing the user's satisfaction with the user interface.

The computing device can be further configured to perform any one of the steps, and/or comprise any one of the features, as described in the previous method steps. This includes, but is not limited to, the ability to handle multi-modal sensor data or data acquired from different sensor types, the use of an assessment algorithm that employs a plurality of different agents, the use of an assessment algorithm that is based on a large language model, the execution of the method as a hidden process simultaneously with and/or during user interface interaction, the automatic stop feature if the determined assessment dataset does not change or does not provide new information content, the semantic analysis of the aggregated data by the assessment algorithm, and the use of a metrics calculation agent by the assessment algorithm.

The computing device can also be configured to use a classification technique to assign a label to data points of the aggregated data, which serve as input features. The classification technique can include Logistic Regression, Decision Trees, Random Forests, Support Vector Machines, and Neural Networks, particularly deep neural networks.

By being configured to perform these steps and/or comprise these features, the computing device can provide a comprehensive and nuanced assessment of the user's satisfaction with the user interface. This can lead to a more accurate and meaningful evaluation, which can in turn inform improvements to the user interface design.

The invention also encompasses a system for user interface testing. This system includes a medical device and a computing device. The medical device can be any device used for medical purposes and/or medical treatment, such as a blood treatment device, a dialysis device, a peritoneal dialysis device, or a plasmapheresis device. The medical device includes a user interface that can be directly implemented in the medical device or implemented in a separate device that exchanges data with the medical device.

The computing device is configured to determine an assessment for the user interface of the medical device. It includes a sensor interface for receiving sensor data during the use of the user interface, a transformer for transforming non-textual sensor data into textual sensor data, an aggregator for aggregating all received sensor data in a textual format, a processing unit for executing an assessment algorithm with the aggregated data to continuously determine an assessment dataset as a result, and a result interface for providing the determined assessment dataset.

The computing device can also be configured to perform additional steps and/or comprise additional features as described in the previous method steps. This can include handling multi-modal sensor data, using an assessment algorithm that employs multiple agents, executing the method as a hidden process, automatically stopping if the assessment dataset does not change or provide new information, performing semantic analysis of the aggregated data, and using a metrics calculation agent.

The system for user interface testing provides a comprehensive solution for assessing the user's satisfaction with the user interface of a medical device. By continuously monitoring and analyzing the user's interaction with the user interface, the system can provide valuable insights that can inform improvements to the user interface design, enhancing its usability and effectiveness.

The invention also includes a computer program product comprising program elements that induce a computing device to carry out the steps of the method for determining an assessment for a user interface. When the program elements are loaded into a memory of the computing device, they enable the device to perform the various steps of the method.

The properties, features and advantages of this invention described above, as well as the manner they are achieved, become clearer and more understandable in the light of the following description and embodiments, which will be described in more detail in the context of the drawings.

This following description does not limit the invention on the contained embodiments. Same components or parts can be labelled with the same reference signs in different figures. In general, the figures are not for scale.

It shall be understood that a preferred embodiment of the present invention can also be any combination of the dependent claims or above embodiments with the respective independent claim.

These and other aspects of the invention will be apparent from and elucidated with reference to the embodiments described hereinafter.

### BRIEF DESCRIPTION OF THE DRAWINGS

- Fig. 1: is a flow chart of a method for determining an assessment for a user interface according to a preferred embodiment of the present invention;
- Fig. 2: is an overview of the structure and architecture of the invention according to a preferred embodiment of the present invention;
- Fig. 3: is a more detailed schematic representation of the assessment algorithm according to a preferred embodiment of the invention;
- Fig. 4: is a schematic representation of using a first LLM;
- Fig. 5: is a schematic representation of using a second LLM.

Any reference signs in the claims should not be construed as limiting the scope.

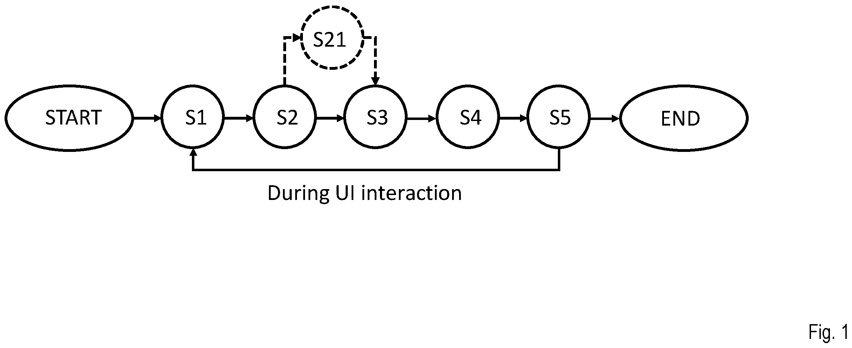

Fig. 1 schematically illustrates an exemplary flowchart for a computer-implemented method for determining an assessment for a user interface UI, in particular in part a graphical UI, GUI, of a medical device MD. The medical device may be a dialyses device.

The method comprises receiving sensor data during use of or interaction with the UI in step S1. The sensor data typically stem from different types of sensors and comprise textual sensor data and non-textual sensor data. Both, the textual and the non-textual sensor data reflect the user's satisfaction with the user interface UI during the course of user interface interaction.

The method comprises transforming the (received) non-textual sensor data in textual sensor data in step S2.

The method may optionally comprise the step S21 in aggregating all received sensor data. Here, the sensor data which have been transformed in textual format and those which have been provided in textual format are aggregated. In case only textual data have been received, it is obviously not necessary to execute step S21, this is why in Fig. 1 this step is represented with dotted lines. Alternatively, also other settings may be relevant, where this step may be omitted and/or skipped to the assessment algorithm.

The method still further comprises a step S3 for feeding the aggregated data in an assessment algorithm S4. The assessment algorithm is configured for continuously determining an assessment dataset as result.

Finally, the method comprises providing the determined assessment dataset on a result interface 15 in step S5.

For instance, the user can enter text on the screen (keyboard) or tap an icon (keyboard, touchscreen). The latter can be used to analyse the user's navigation behaviour. If the number of menus is relatively small, with a few predefined options from which the user selects a combination, the amount of information to be gained is manageable and can be stored in the system in the form of predefined evaluations, for example:
- User selects a smiling smiley from a list of smileys: "User likes the function";
- User opens menu A, suddenly closes menu A to open it again: "User cannot find what they are looking for in menu A";

- User opens menu A, makes a selection in menu A, closes menu A, opens menu A again and cancels the selection in menu A: "User is unsure about the selection in menu A";
- User opens menu A, closes it again abruptly and returns to menu A with a time delay and makes a selection there: "Selection in menu A is difficult"; and/or
- User selects option A.a in menu A, then option C.b in menu C and goes back to cancel the selection of option A.a: "Task distribution between menus A and C is unclear", if menu A and menu C are recognisably related,
- etc.

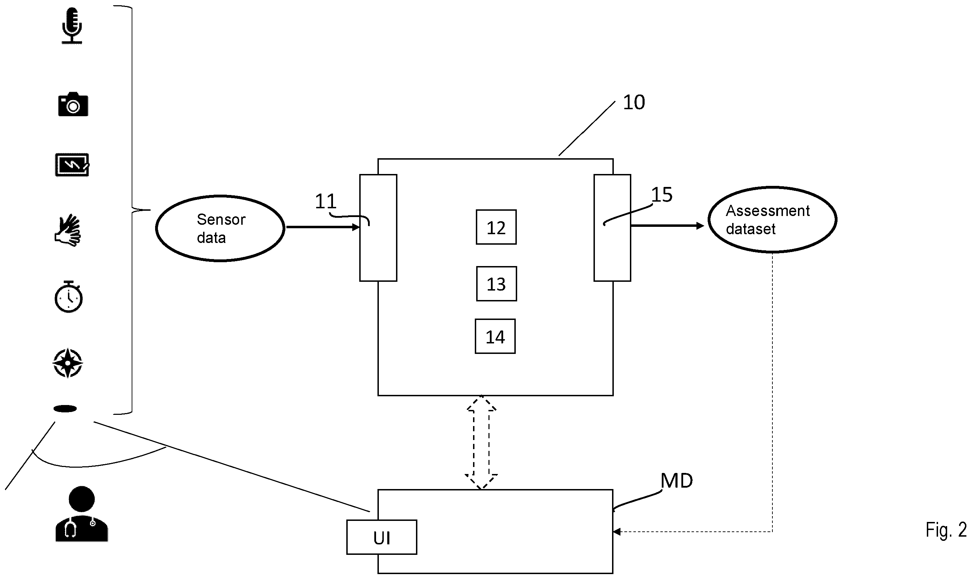

Fig. 2 schematically illustrates an exemplary architecture of a computing device 10 for determination of an assessment for a user interface UI (also in short simply: UI) of a medical device MD. As represented in Fig. 2, the user is operating a user interface UI of the medical device. The UI may be part of the medical device and/or may be part of a separate device which is data connection with all devices and modules, like e.g., a mobile device.

The computing device 10 comprises a sensor interface 11 for receiving sensor data from different types of sensors during use of the UI or during the course of UI interaction. The user's UI interaction is observed and detected by the different sensors, schematically represented in Fig. 2 with the different pictograms on the left side. The sensors comprise virtual sensors which acquire textual sensor data and physical sensors which acquire non-textual sensor data (like measurements), wherein both the textual and the non-textual sensor data reflect the user's satisfaction with the user interface during the course of user interface interaction.

The computing device 10 may comprise a transformer 12 which is an electronic module and which is configured for transforming the non-textual sensor data in textual sensor data. It serves as translator for translating sensor data in textual sensor data.

The computing device 10 may further optionally comprise an aggregator 13 which is also an electronic module and is configured for aggregating all received sensor data which are provided or have been transformed in a textual format.

The computing device 10 further comprises a processing unit 14 which is configured for executing an assessment algorithm S4 with the aggregated data for continuously determining an assessment dataset as result.

The computing device 10 still further comprises a result interface 15 for providing the determined assessment dataset.

The result interface 15 may comprise a user interface for graphical or textual representation of the determined assessment dataset. This may be used for user verification of the computationally determined assessment dataset. Later and after the verification the result may be used for automatic tuning or improvement of the UI. The result interface 15 may therefore comprise a data interface to a UI developer tool for automatically triggering changes to UI design.

The computing device 10 may be configured for performing the method for determining an assessment dataset for UI assessment as described above.

A system may comprise the computing device 10 and the medical device MD.

The computing device 10 may be in data exchange with the medical device MD. Alternatively, the computing device 10 may be part of the medical device MD (not shown in Fig. 2). As depicted with dashed line in Fig. 2, the assessment dataset may be used in a downstream task to amend the functionality of the medical device MD by adapting the UI thereof.

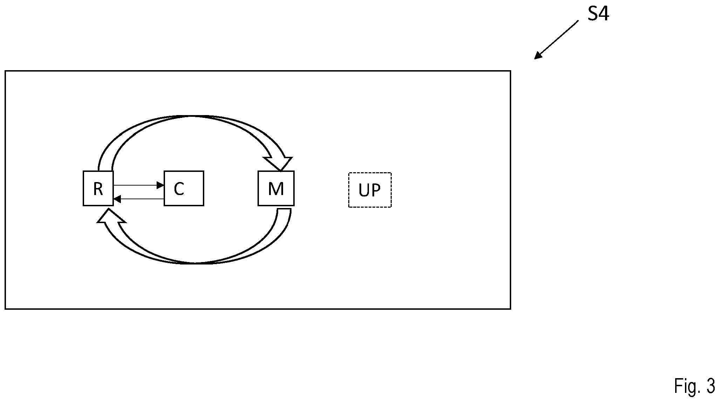

Fig. 3 represents an optional embodiment of the assessment algorithm S4. The assessment algorithm may be based on basic data driven tools using machine learning. The assessment algorithm may comprise a research agent R, a critic agent C, a metrics calculation agent M and a user proxy agent UP.

The research agent R is configured for generating themes/ topics and/or for text classification. In particular, the research agent R is configured to "read" (analyse) the uploaded text and defines a list of all themes along with a relevant example drawn from the text.

The critic agent C is configured for evaluating the completeness and/or the semantic sensibility of the researcher's themes/topics generation and/or classification. The metrics calculation agent M is configured for evaluating the quality of the themes/topics generation made by the research agent R. The metrics calculation agent M may use Coverage, Affinity, and Relevance to evaluate the quality of the theme extraction. The Critic Agent searches for themes not identified by the researcher R and declares whether the result of the researcher R is correct. If it is not, the work of the researcher R is recursively corrected until the critic agent C approves it or until the maximum number of iterations (defined by the user) is reached.

In a second step, the researcher R may create categories and subcategories to optimally classify the themes. In a verification of this second step, the critic agent C evaluates the completeness of the classification and the semantic sensibility of the categories.

In a third step, the researcher R may abstract the list of themes into a list of main themes and comments.

In a last step, the critic agent C reviews the results from the previous step and creates a unified document mediating between the two different analyses. The presence of at least two separate and independent agents R, C in the analysis, along with recursive correction and metric calculations, ensures the completeness of the analysis.

Optionally, as indicated in Fig. 3 by dashed lines, a user proxy agent UP may be configured as pseudo interface between the assessment algorithm S4 and the user and/or may mediate the user's requests and needs to the algorithmic functionality.

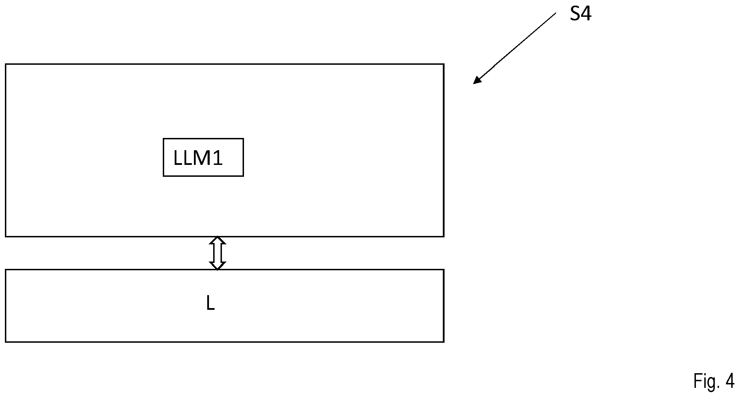

As represented in Fig. 4, the assessment algorithm may use a first LLM1 for determining the assessment dataset based on the received (textual and non-textual) sensor data.

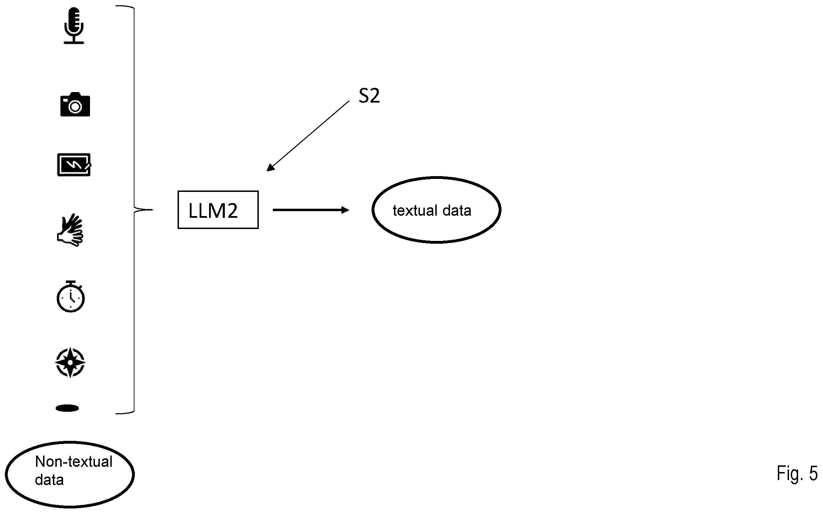

The step S2 of transforming non-textual sensor data into textual sensor data may make use of a second LLM2 as shown in Fig. 5.

Wherever not already described explicitly, individual embodiments, or their individual aspects and features, described in relation to the drawings can be combined or exchanged with one another without limiting or widening the scope of the described invention, whenever such a combination or exchange is meaningful and in the sense of this invention. Advantages which are described with respect to a particular embodiment of present invention or with respect to a particular figure are, wherever applicable, also advantages of other embodiments of the present invention.

### List of Reference Signs

- S1: Step of receiving sensor data
- S2: Step of transforming non-textual sensor data in textual sensor data
- S21: Step of aggregting received sensor data
- S3: Step of feeding the aggregated sensor dta in assessment algorithm
- S4: Step of executing the assessment algorithm
- S5: Step of providing the determined assessment dataset on a result interface

- MD: Medical device
- UI: User interface of the medical device
- 10: Computing device
- 11: Sensor interface
- 12: Transformer
- 13: Aggregator
- 14: Processing unit
- 15: Result interface

- LLM1: First Large Language Model
- LLM2: Second Large Language Model

- R: Research Agent
- C: Critic Agent
- M: Metrics Calculation Agent
- UP: User Proxy Agent

## Claims

1. Computer-implemented method for determining an assessment for a user interface (UI) of a medical device (MD), comprising the method steps of:
- Receiving (S1) sensor data during use of the UI, comprising textual sensor data and non-textual sensor data, wherein the sensor data reflect the user's satisfaction with the user interface during the course of user interface interaction;
- Transforming (S2) the non-textual sensor data in textual sensor data;
- Aggregating (S21) all received sensor data in a textual format;
- Feeding (S3) the aggregated data in an assessment algorithm (S4) for continuously determining an assessment dataset as result;
- Providing (S5) the determined assessment dataset on a result interface.

2. Method according to claim 1, wherein the sensor data are multi-modal and/or are acquired on different sensor types, comprising a time-measurement sensor, a gesture sensor for gesture recognition, wired glove sensor, acoustic sensor, in particular a microphone, input on a user interface, virtual sensor data, and/or optical sensor.

3. Method according to any of the preceding claims, wherein the assessment algorithm is based on a first large language model (LLM1), which was trained to provide an assessment result for textual input.

4. Method according to any of the preceding claims, wherein the step of transforming comprises to use a second large language model (LLM2), which was trained to transform non-textual data into textual data.

5. Method according to any of the preceding claims, wherein the method is executed as hidden process simultaneously with and/or during user interface interaction.

6. Method according to any of the preceding claims, wherein the method automatically stops if the determined assessment dataset does not change and/or does not provide new information content, wherein the fact that no new information is provided is based on an entropy-based algorithm and/or on testing of generation of new categories or subcategories.

7. Method according to any of the preceding claims, wherein the assessment algorithm (S4) analyses the aggregated data with regard to its semantic content.

8. Method according to any of the preceding claims, wherein the assessment algorithm (S4) uses a metrics calculation agent (M) which is responsible for calculating metrics for text analysis, in particular for classification.

9. Method according to any of the preceding claims, wherein the assessment algorithm (S4) makes use of a plurality of different agents, at least comprising a research agent (R) and/or a critic agent (C), which are configured for analysing the received sensor data to provide an assessment, and wherein the research agent (R) provides a first result and the critic agent (C) provides a second result and a reconciliation algorithm is applied on the first and second result to provide a final result.

10. Method according to any of the preceding claims, wherein the assessment algorithm (S4) comprises a research agent (R) which is responsible for text classification and a critic agent (C) which is responsible for evaluating the completeness and/or the semantic sensibility of the researcher's classification.

11. Method according to the directly preceding claim, wherein research agent (R) makes use of a classification technique to assign a (class) label to data points of the aggregated data which serve as input features.

12. Computing device (10) for determination of an assessment for a user interface, UI, of a medical device (MD), comprising:
- Sensor interface (11) for receiving sensor data from sensors during use of the UI, wherein the sensors comprise virtual sensors which acquire textual sensor data and physical sensors which acquire non-textual sensor data, wherein the sensor data reflect the user's satisfaction with the user interface during the course of user interface interaction;
- A transformer (12) which is configured for transforming the non-textual sensor data in textual sensor data;
- An aggregator (13) for aggregating all received sensor data in a textual format;
- A processing unit (14) which is configured for executing an assessment algorithm (S4) with the aggregated data for continuously determining an assessment dataset as result;
- A result interface (15) for providing the determined assessment dataset.

13. Computing device (10) according to the directly preceding claim, further configured to perform any one of the steps, and/or comprise any one of the features, of any one of the method claims 2 to 11.

14. System for user interface testing, comprising a medical device (MD) and a computing device (10) according to claims 12 or 13.

15. A computer program product comprising program elements which induce a computing device (10) to carry out the steps of the method for determining an assessment for a user interface according to any of the of the preceding method claims, when the program elements are loaded into a memory of the computing device (10).
